# EUROPEAN PATENT SPECIFICATION

(11) **EP 1 358 318 B1**
(45) Date of publication and mention of the grant of the patent: **23.08.2006**
(21) Application number: 02704692.9
(22) Date of filing: 07.02.2002
(51) Int. Cl.: C12N 5/20, C12N 1/19, C12N 1/21, C12N 5/10, C07K 16/30, A61K 39/395, A61K 51/10, A61K 47/48, A61P 35/00

(54) **HYBRIDOMA CELL LINE G250 AND ITS USE FOR PRODUCING MONOCLONAL ANTIBODIES**
HYBRIDOMZELLLINIE G250 UND DEREN VERWENDUNG ZUR HERSTELLUNG MONOKLONALER ANTIKÖRPER
LIGNEE DE CELLULES D'HYBRIDOMES G250 ET SON UTILISATION POUR PRODUIRE DES ANTICORPS MONOCLONAUX

(30) Priority: 07.02.2001 US 266853 P; 05.10.2001 US 327008 P
(43) Date of publication of application: 05.11.2003
(60) Divisional application: 06014768.3
(73) Proprietor: Wilex AG, 81675 München (DE)
(72) Inventor: OOSTERWIJK, Egbert, NL-6641 Beuningen (NL); WARNAAR, Sven, NL-2334 Leiden (NL); ULLRICH, Stefan, 82319 Stamberg (DE)
(74) Representative: Weiss, Wolfgang
(86) International application number: PCT/EP2002/001282
(87) International publication number: WO 2002/062972

(56) References cited:
- EP-A- 0 210 970
- WO-A-00/67792
- WO-A-88/08854
- WO-A-99/54342
- E. OOSTERWIJK ET AL.: "Monoclonal antibody G250 recognizes a determinant present in renal cell carcinoma and absent from normal kidney." INTERNATIONAL JOURNAL OF CANCER, vol. 38, 1986, pages 489-494, XP008009792 Copenhagen, DK cited in the application

## Description

The invention relates to the hybridoma cell G250 or any progeny cell thereof capable of producing G250 antibody.

The fusion of mouse myeloma cells with the spleen cells from immunized mice, first described by Kohler and Milstein, Nature, 256, 495-997 (1975), makes it possible to obtain continuous cell lines which produce homogeneous antibodies, referred to as monoclonal antibodies (mAb).

Many examples exist where hybrid cells or hybridomas are described. These hybridomas are used to produce antibodies useful for various scientific investigations (Current Topics in Microbiology and Immunology, volume 81 - "Lymphocyte Hybridomas", F. Melchers et al., Springer-Verlag (1978) and references therein; C.J. Barnstable et al., Cell, (1978), 14, 9-20; P. Parham, W.F. Bodmer, Nature (1978), 276, 397-399; Handbook of Experimental Immunology, 3rd edition, vol. 2, D.M. Wier, editor, Blackwell, 1978, Chapter 25, Chem. Eng. News, 15-17 (1979); Kennett, R.H., McKearn, J.T., and Bechtol, K.B. (1980) Monoclonal Antibodies. Hybridomas: A New Dimension in Biological Analysis (Plenum, New York)). These reports describe the principal techniques for the production of monoclonal antibodies by hybridomas.

The monoclonal antibody G250, subclass IgG1, recognizes an antigen preferentially expressed on membranes of renal cell carcinoma cells (RCC) and not expressed in normal proximal tubular epithelium. The antibody G250 was obtained by immunizing a mouse with cell homogenates from primary RCC lesions obtained from different patients (Oosterwijk et al., Int. J. Cancer 38 (1986), 489-494).

The monoclonal antibody G250 as well as chimeric derivatives thereof have been used in clinical studies (Steffens et al., J. Clin. Oncol. 15 (1997), 1529-1537). The nucleic acid sequence coding for the antigen-binding site of G250 is subject matter of co-pending US application No. 60/266 853, which is herein incorporated by reference.

The production of a hybridoma cell line expressing G250 antibody was generally described in the international patent application WO88/08854 and Oosterwijk et al. (supra). As stated above, a cell homogenate from primary RCC lesions obtained from different patients and thus an unspecific material was used as an immunogen. Furthermore, the hybridoma cell line had not been deposited with a recognized depository institution according to the Budapest Treaty. Thus, an exact reproduction of the G250 hybridoma cell line from the publically available prior art documents does not seem to be possible.

Meanwhile it has been found that the G250 antibody binds to the so-called MN antigen which is described e.g. in WO93/18152. The G250 binding site on the MN antigen is, however, not known at present. Moreover, recent results show that the G250 binding site is a conformational epitope which further increases the burden to reproduce the G250 hybridoma cell line, since no specified epitope sequence on the MN antigen which binds to G250 can be provided.

Thus, the present invention relates to a hybridoma cell capable of producing a G250 monoclonal antibody. This hybridoma cell was deposited under the Budapest Treaty for the Deposit of Microorganisms on September 11, 2001 at Deutsche Sammlung von Mikroorganismen und Zellkulturen GmbH (DSMZ), Mascheroder Weg 1 b, 38124 Braunschweig, Germany under the Accession Number DSM ACC 2526. The deposit is the first publically available disclosure of a G250 antibody producing hybridoma cell line.

A further aspect of the invention is a progeny cell derived from said hybridoma DSM ACC 2526 which produces a G250 antibody.

In a preferred embodiment, the present invention relates to a progeny cell of the deposited hybridoma cell producing a G250 antibody wherein the progeny cell is obtained by recombinant DNA methods, e.g. by transferring genetic material encoding the G250 antibody or at least the antigen-binding site thereof into a receptor cell. The genetic material may be directly or indirectly obtained from the deposited hybridoma cell G250. "Directly obtained" means that the G250 genetic material is derived from the deposited hybridoma cell. "Indirectly obtained" means that the G250 genetic material is derived from an already existing G250 progeny cell or from other sources including chemical synthesis.

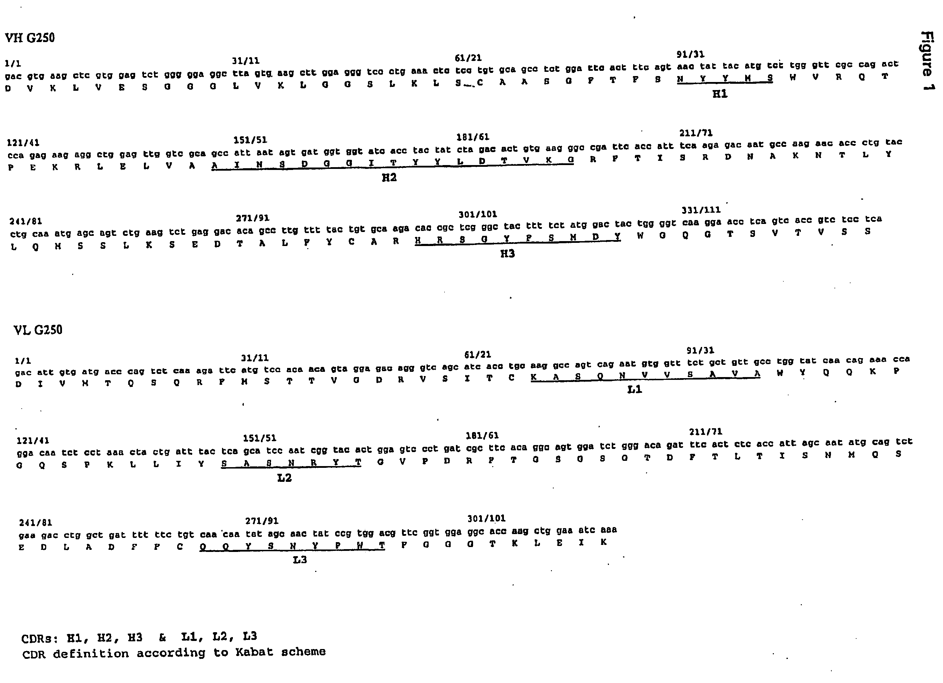

Preferably the G250 genetic material comprises nucleotide sequences encoding at least the G250 antigen-binding site, particularly nucleotide sequences encoding the complementary determining regions CDR3, CDR2 and/or CDR1 of the heavy chain antigen-binding site as shown in Figure 1 (designated H1-H3) and/or the complementary determining regions CDR3, CDR2 and/or CDR1 of the light chain antigen-binding site (designated L1-L3) as shown in Figure 1.

According to the invention the term "G250 antibody" covers any antibody including multispecific antibodies (e.g. bispecific antibodies) and antibody fragments as long as they exhibit the desired activity, i.e. at least one G250 antigen-binding site. The antibody may be an IgM, IgG (e.g. IgG₁, IgG₂, IgG₃ or IgG₄), IgD, IgA or IgE, particularly IgG antibody, a recombinant antibody or an antibody fragment obtained by proteolytic methods or by recombinant DNA methods.

The term "monoclonal antibody" as used herein refers to an antibody obtained from a population of substantially homogeneous antibodies, i.e., the individual antibodies comprising the population are identical, except for possibly naturally occurring mutations that may be present in minor amounts.

The term "antibody" as used herein refers to any polypeptide containing at least one G250 antigen-binding site, i.e. at least the CDR3 region of the G250 heavy chain and/or the CDR3 region of the G250 light chain or a variant G250 CDR3 region having an identity of at least 80%, preferably at least 90% to the original G250 CDR3 region on the amino acid level, provided that the variant CDR3 region has equivalent antigen-binding characteristics, particularly affinity and specificity compared to the original CDR3 region.

Preferably, the term "antibody" herein includes chimeric antibodies, humanized and fully humanized antibodies, single chain antibodies, e.g. sFv antibody fragments, diabody fragments, proteolytic or recombinant antibody fragments such as Fv-, Fab-, Fab'- or F(ab')₂-fragments or other antigen-binding subsequences of antibodies. The antibody may also be a fusion or a conjugate with other entities.

The antibodies herein specifically include chimeric antibodies in which a portion of the heavy and/or light chain including the antigen-binding site is identical with or homologous to corresponding sequences derived from the original hybridoma cell line G250, while the remainder of the chains is identical with or homologous to corresponding sequences derived from other species or belonging to another antibody class or subclass as well as fragments of such antibodies as long as they exhibit the desired biological activity. More preferably, the chimeric antibody comprises variable regions, e.g. the complement-determining regions (CDRs) and and the framework regions from the heavy chain and the light chain of the original G250 monoclonal antibody and constant human sequences, particularly constant human kappa light chain and gamma heavy chain sequences. The manufacture of chimeric antibodies is described e.g., by Morrison et al. (Proc. Natl. Acad. Sci. USA 81 (1984), 6851-6855), which is herein incorporated by reference.

Further, antibody herein specifically includes humanized antibodies or fully human antibodies. Humanized antibodies are immunoglobulins, immunoglobulin chains or fragments thereof which contain minimal sequence derived from non-human immunoglobulin. More particularly, humanized antibodies are human immunoglobulins in which residues from a CDR of a given human antibody are replaced by residues from the G250 CDR, particularly the CDR1, 2 and/or 3 region of the heavy and/or light chain. Furthermore, humanized antibodies may comprise residues which are found neither in the recipient human antibody, nor in the imported G250 CDR sequences. These modifications are made to further refine and optimize antibody performance. In general, the humanized or fully human antibody will comprise substantially all of at least 1, and typically 2, variable domains, in which all or substantially all of the CDR regions correspond to those of the original G250 immunoglobulin and all or substantially all of the framework regions and constant regions are those of a human immunoglobulin sequence. The manufacture of humanized antibodies is described, e.g. in Jones et al. (Nature 321 (1986), 522-525), Riechmann et al. (Nature 332 (1988), 323-329 and Presta (Curr. Op. Struct. Biol. 2 (1992), 332-339), which are herein incorporated by reference.

Further, antibodies specifically include single-chain antibodies such as single-chain Fv antibody fragments comprising the VH and VL domains of an antibody, wherein these domains are present in a single polypeptide chain. Generally, the Fv polypeptide further comprises a polypeptide linker between the VH and VL domains which enables the sFv to form the desired structure for antigen binding. The manufacture of sFv antibodies is described e.g. by Plückthun in: The Pharmacology of Monoclonal Antibodies, Vol. 113, Rosenburg and Moore, Eds., Springer Verlag, NY, pp. 269-315 (1994), Barbas III (Methods: Companion Methods Enzymol. 2 (1991), 119) and Hoogenboom et al. (Immunol. Rev. 130 (1992), 41-68), which are herein incorporated by reference.

Further, antibodies specifically include diabodies, i.e small antibody fragments with two antigen-binding sites, which fragments comprise a heavy chain variable domain connected to a light chain variable domain in the same polypeptide chain. By using a linker that is too short to allow pairing between the two domains on the same chain, the domains are forced to pair with the complementary domains of another chain and create two antigen binding sites. The manufacture of diabodies is described e.g. by Hollinger et al., (Proc. Natl. Acad. Sci. USA 90 (1993), 6444-6448), which is herein incorporated by reference.

Further, antibodies specifically include multispecific antibodies e.g. heterobispecific antibodies comprising at least one G250 antigen-binding site and the antigen-binding site from a different antibody, e.g. an anti-CD3-antibody.

The antibody produced by the G250 hybridoma cell or a progeny cell thereof may be fused or coupled to a marker or effector component. For example, the G250 antibody may be recombinantly modified so that it is linked to cytokines such as interleukin-2 (IL-2), tumor necrosis factor (TNF) and/or granulocyte macrophage colony stimulating factor (GM-CSF).

Furthermore, the G250 antibody may be conjugated, e.g. by covalent coupling or fused to a suitable marker group, e.g. a fluorescent group, a radioactive marker group etc. or a cytotoxic agent including radioactive isotopes such as I, Y, Pr, Tm, In, such as ¹²³I, ¹²⁵I, ¹³⁵I, ^{99m}Tm or ¹¹¹In, chemotherapeutic agents and toxins such as enzymatically active toxins of bacterial, fungal, plant or animal origin, or fragments thereof.

The receptor cell into which G250 specific genetic material from the original G250 hybridoma cell or any progeny thereof or of synthetic origin is transferred may be any suitable host cell capable of expressing antibodies. For example, the host cell maybe a prokaryotic cell, preferably an E. coli cell or a eukaryotic cell. Preferred eukaryotic cells are e.g. insect, yeast and mammalian cells. Most preferred host cells are mammalian cells, e.g. human or mouse myeloma cells or CHO-cells.

Further, the present invention comprises a method of producing G250 antibody or derivatives thereof comprising:
cultivating a G250 hybridoma cell or a progeny cell thereof under suitable conditions, wherein a G250 antibody is produced and obtaining the antibody and/or derivative thereof from the cell and/or from the cell culture medium.

The antibody obtained according to this method is particularly useful for producing pharmaceutical formulations which comprise the antibody as an active agent, besides pharmaceutically acceptable carriers, diluents and/or adjuvants.

In an especially preferred embodiment, the active agent is a chimeric G250 antibody which may be present as such or as a conjugate with a radioactive group, e.g. with ¹²³I, ¹²⁵I, ¹³¹I, ^{99m}Tm or ¹¹¹In.

Further, the present invention relates to the use of the deposited hybridoma cell and progeny cells thereof for manufacturing G250 antibodies as described above, e.g. monoclonal antibodies, chimeric antibodies, humanized antibodies, fully humanized antibodies, bispecific antibodies and antibody fragments such as (Fab')₂-, Fab'-, Fab- and Fv-fragments.

The chimeric G250 antibody was successfully used in clinical studies for the treatment of renal cell carcinoma patients after surgery. Even though the study has not yet been completed, the treated patients already show a significant increase in median survival (more than 15 months) compared to untreated patients (three months) or patients treated with standard therapy (10-12 months).

Surprisingly, it was found that in some cases tumor regression occurred more than six months after start of therapy. Thus, chimeric G250 antibody and other G250 antibodies are capable of eliciting a delayed immune response in cancer therapy, preferably in the treatment of renal cell carcinoma and more preferably for the treatment of metastases after tumor surgery.

### Example 1

### Deposit of the G250 hybridoma cell line

The G250 hybridoma cell line was produced as described in Example 1 of WO88/08854. Therein a general immunization protocol is given. Further informations, e.g. a molecular characterization of the G250 antibody and the G250 hybridoma cell are lacking.

The G250 hybridoma cell line was deposited according to the requirements of the Budapest Treaty at DSMZ under the accession No. DSM ACC 2526 on September 11, 2001.

### Example 2

### Mapping of the epitope recognized by monoclonal antibody G250

From collective experimental evidence it is assumed that the G250 protein epitope recognized by monoclonal antibody G250 (MAbG250) is conformational, most likely of a non-linear nature. This assumption is based on the following observations:
- MabG250 does not react with antigen G250 in Western blot analysis
- Purification of G250 antigen by MAbG250 affinity chromatography is highly inefficient, with efficiencies decreasing with increased time needed to perform the purification, suggesting rapid degradation/ unfolding of the G250 epitope, in spite of optimal conditions to prevent proteolysis.
- Addition of detergents in general lead to decreased G250 detection levels in ELISA
- Truncation of the cDNA encoding for G250 antigen, followed by transient transfection in G250-negative cells, followed by immunohistochemical analysis revealed:

| cDNA construct | G250-reactivity |
|---|---|
| nt 1-1500 (aa 1-459), complete protein | + |
| nt 1-1218 (aa 1-406), G250 without transmembrane region | +/- |
| nt 1-1074 (aa 1-358) | - |
| nt 1-843 (aa 1-281) | - |
| nt 1-672 (aa 1-224) | - |
| nt 1-450 (aa 1-150) | - |

It was found that G250-reactivity was lost, even when large cDNA constructs were used.

To further delineate the G250 epitope, the NovaTope system (Novagen Inc.) was used, a system which ensures expression of all constructs ligated into this vector, i.e., all possible fragments are expressed, irrespective of reading frame. This allows identification of epitopes present in the middle part of a protein.

The cDNA encoding for the MN (G250) antigen was digested with DNAse I for 2 hours, resulting in fragments of approximately 50-200 nucleotides encoding polypeptides having a length of about 15-70 amino acids. After dA tailing, the fragments were inserted into the pScreen T vector, and the ligation mixture was added to competent E.coli DE3 bacteria for transformation. Bacteria were plated on selection plates. Bacterial clones were blotted onto nitrocellulose filters and screened with G250 antibody. In total 7 colonies were identified with MAbG250. G250 reactivity was weak. After further re-screening, only one bacterial clone appeared to react with MAbG250, albeit extraordinary very weak, and most likely due to background staining. Sequence analysis of the clone did not reveal any G250-sequences, but showed the presence of a truncated vector. Thus, the staining of the nitrocellulose blot was most likely the result of nonspecific background.

In a further experiment, cDNA encoding the MN (G250) antigen was digested with DNAse I, but now fragments of 200 - 600 nucleotides, encoding polypeptides of approximately 65 - 200 amino acids were isolated and cloned into the expression vector and expressed in bacteria. Separate bacterial colonies were screened for G250 reactivity.

Reactivity with G250 was not apparent. Blots were stained for extended times, after which a faint staining was observed with 6 bacterial isolates. The bacterial isolates only contained a truncated vector and an unrelated sequence.

Thus, no partial sequence of the MN antigen having strong reactivity with MAbG250 could be identified.

These results strongly suggest that the G250 epitope is a conformational epitope. Further, the G250 epitope on the MN antigen cannot be characterized by conventional methods even with considerable efforts.

### Example 3

Development of a chimeric G250 IgG production cell line

### General strategy

In order to construct a mouse/human chimeric version of the murine G250 antibody, the variable region genes for the heavy and light chains, which determine the binding specificity of the antibody, were cloned from the G250 murine hybridoma and assembled in vitro with human constant region genes to generate mouse/human chimeric antibody genes. Expression of these chimeric genes in the appropriate cell line resulted in production of a chimeric antibody with the same binding characteristics as the original murine antibody, but with approximately 75% of the molecule comprising human sequences.

The strategy for cloning the variable regions for the heavy and light chain genes from the G250 hybridoma was based upon the linkage in the genome between the variable region and the corresponding J (joining) region for functionally rearranged (and expressed) immunoglobulin genes. J region DNA probes can be used to screen genomic libraries to isolate DNA linked to the J regions; DNA in the germline configuration (unrearranged) would also hybridize to J probes, but is not linked to a variable region sequence and can be identified by restriction enzyme analysis enzyme analysis of the isolated clones.

The cloning strategy, therefore, was to isolate variable regions from rearranged heavy and light chain genes using JH and JK probes. In addition, to assist in identifying and characterizing the correct genomic clones, cDNA clones corresponding to the heavy and light chain variable regions were also obtained from the mRNA produced in the G250 hybridoma. Those genomic clones that matched the G250 cDNA sequences were cloned into expression vectors containing human constant regions and transfected into mouse myeloma cells to determine if an antibody was produced. The antibody from producing cells was then tested for binding specificity compared to the murine G250 antibody.

### Cloning of G250 VH and VL cDNA

Total RNA was isolated from G250 hybridoma cell and used to generate specific VH and VL cDNA clones. First strand cDNA synthesis was performed using oligonucleotide primers specific for the murine kappa and IgG constant regions. After dG tailing, second strand synthesis was performed using C-tailed oligonucleotide primers. The heavy and light chain variable regions were amplified via polymerase chain reaction using specific 5' and 3' primers, and the amplification products were cloned into the plasmid vector pUC19. VH and VL clones were subjected to DNA sequence analysis to establish the base sequences of the putative G250 heavy and light chain variable regions. The sequencing results are shown in Fig. 1.

### Cloning of the G250 Variable Region Genes:

To clone the variable region genes, high molecular weight genomic DNA was isolated from G250 hybridoma cells by treatment of isolated nuclei with SDS and proteinase K followed by two phenol extractions and an ethanol precipitation.

Southern blot analysis using a J region probe for the heavy chain locus suggested that the G250 heavy chain was contained on a 2.3 kilobase (Kb) Eco RI DNA fragment. Accordingly, the Eco RI fragments from hybridoma DNA were fractionated on a 0.8% agarose gel and the size range of approximately 2-3 Kb fragments was eluted from the gel and ligated to the vector arms of the lambda bacteriophage vector Zap II (Stratagene, La Jolla California, USA). The ligation was packaged into phage particles in vitro using Gigapack Gold (Stratagene) and plated on E.coli LE392 cells at a density of approximately 20.000 plaques per 150 mm plate. The plaques were transferred to nitrocellulose filters and probed with a ³²P-labeled DNA probe (a 2.0 Kb Eco RI-BamHI fragment containing the murine J3 and J4 exons).

For the light chain G250 variable region gene, a Southern blot analysis using a murine J region probe from the kappa locus indicated that the correct gene was located on a 5.5 Kb HindIII fragment. Accordingly, G250 hybridoma DNA was digested with HindIII and DNA fragments of 5-6 Kb were isolated from a 0.8% agarose gel and ligated to the arms of the bacteriophage lambda vector Charon 27. The ligated DNA was packaged into phage particles in vitro using Gigapack Gold (Stratagene), and plated on E.coli LE392 cells at a density of approximately 20.000 plaques per 150 mm plate. The plaques were transferred to nitrocellulose filters and probed with a ³²P-labeled DNA probe (a 2.7 Kb HindIII fragment containing all five J kappa exons).

Positive clones for the heavy and light chain genes were isolated following at least 3 rounds of plaque purification using the J region probes to verify hybridization to the phage DNA at each stage of purification. Purified DNA from the isolated phage clones for the heavy and light genes was digested with EcoRI (heavy chain) or Hindlll (light chain) and fractionated on agarose gels. The appropriate size fragments (2.3 Kb for the heavy chain and 5.5 Kb for the light chain) were isolated from the gels, and subcloned into the plasmid vector pUC19. These subcloned DNA fragments were shown by restriction endonuclease mapping and DNA sequence analysis to contain variable region antibody genes that matched the structures of the cDNA clones originally obtained from the G250 hybridoma.

### Expression Plasmids

The 2.3 Kb Eco RI heavy chain variable region fragment was cloned into a suitable expression vector which contains the human G1 constant region and a gene which confers resistance to a selectable marker in mammalian cells. The 5.5. Kb HindIII light chain fragment was cloned into a suitable expression vector which contains the human kappa constant region and a gene conferring resistance to a selectable marker in mammalian cells. The presence of the cloned fragments and their orientation in the expression vectors was determined by restriction enzyme mapping. The basic vectors have been used previously for the construction of chimeric antibodies (Sun, L. et al., (1987) PNAS 84, p. 214; Knight et al., (1993), Molecular Immunology 30, p. 1443-1453). Expression of the chimeric antibody genes is achieved using the natural immunoglobulin promoters present upstream of the cloned variable regions; downstream regulatory signals are provided by the elements associated with the constant region genes.

### Expression of the G250 Chimeric Antibody

The heavy and light chain expression vectors were used to co-transfect a non-producing mouse myeloma cell line using the technique of electroporation. An ELISA assay was used to screen clones for secreted antibody containing a human Fc region, and the highest producer (cG250) was chosen for further characterization.

The chimeric antibody was purified by chromatography on protein A-sepharose, and analyzed in a competition assay to verify that the antibody has the correct specificity. The cG250 antibody was found to be equally effective in competing with ¹²⁵I-labelled murine G250 for binding to A704 renal cell carcinoma cells (which express the G250 antigen) as the unlabeled murine G250 antibody. The chimeric G250 antibody, therefore, is similar in its binding characteristics to the original murine G250 antibody.

### Example 4

Use of chimeric G250 antibody in a clinical study

### Administration protocol

Renal cell carcinoma patients, having metastases after surgery, were treated by administering 50 mg chimeric G250 antibody via 30 min. infusion once per week for 12 weeks.

### Efficacy conclusions:

In the present study durable stable disease for over six months was achieved in eight out of 32 patients (25%) of the patients. The median time to progression for all patients was 16 weeks (mean 26,67 weeks) with a range from 4 to 70 weeks. As of December 2001, the date of cut off, five patients are still free of progression (51 +, 56+, 60+, 64+, and 70 + weeks).

The importance of these findings is supported by the fact that most of these patients had documented progressive disease, for another two patients it is assumed that they were progressive at study entry. In addition, three of these patients were refractory to prior cytokine- and chemotherapy or had relapsed several years after cytokine treatment. Four patients had no prior therapy and were treated with the study drug immediately after diagnosis of metastases. At study entry three of these patients had a Karnofsky performance status (KPS) of 80%, two a KPS of 90%. Hemoglobin (Hgb) was below 10 g/dL in 5 patients. The median performance status of 90% (mean 91%) is identical to the group of patients who have not responded. The median Hgb of 9.8 g/dL (mean 10.97 g/dL) is also relatively low.

One patient achieved a complete objective remission. The objective response occurred late, more than six months after start of study therapy. Another patient experienced a relevant reduction of 59% in size of his target lesions. The response of both patients is ongoing at week 64 and 70, respectively, as evaluated in December 2001. The tumor regression occurred late, more than six months after start of study therapy. An explanation for the late response may be that the treatment duration with iv injections of the chimeric antibody was relatively short and a delayed immune response unrelated to ADCC developed.

The response is durable at more than one year and is still ongoing December 2001. The overall rate of clinical benefit (at least more than six months or objective response) is 25%. In addition it should be noted that at six months after start of study treatment, 87.5% (28/32) of the patients were still alive.

At the end of November 2001, an estimation of median survival was calculated to be at least 13.5 months.

At the end of January 2002, an estimation of median survival was calculated to be at least 15 months. This is significantly higher than the median survival of untreated patients (three months) and the median survival of patients treated with an FDA approved standard therapy, i.e. administration of high dose interleukin 2 (10-12 months).

Thus, it becomes evident that chimeric antibody G250 is a suitable therapeutic agent for the treatment of renal cell carcinoma. In a preferred embodiment the treatment comprises a surgical removal of the main tumor and a subsequent administration of G250, in order to eliminate metastases distributed throughout the body.

## Claims

1. Hybridoma cell DSM ACC 2526 which produces the monoclonal antibody G250.

2. Use of the hybridoma cell according to claim 1 for the preparation of a progeny cell which produces the monoclonal antibody G250.

3. The use according to claim 2, wherein said progeny cell has been obtained by transfer of genetic material encoding antibody G250 or at least the antigen-binding site thereof into a receptor cell.

4. The use according to claim 2 or 3, wherein said progeny cell produces a chimeric antibody, a humanized antibody, a fully humanized antibody, a bispecific antibody, a single chain antibody, or F(ab')₂, Fab', or Fab antibody fragments.

5. The use according to any one of claims 2 to 4, wherein said progeny cell is a prokaryotic cell.

6. The use according to claim 5, wherein said progeny cell is an E. coli cell.

7. The use according to any one of claims 2 to 4, wherein said progeny cell is a eukaryotic cell.

8. The use according to claim 7, wherein said progeny cell is a yeast cell, a myeloma cell or a CHO-cell.

9. A method of producing a G250 antibody comprising:
cultivating a cell according to any one of claims 1-7 under suitable conditions, wherein the antibody is produced and obtaining the antibody from the cell and/or from the culture medium.

10. The method according to claim 9 used for producing a pharmaceutical formulation which comprises the G250 antibody as an active agent.

11. The method according to claim 10, wherein the pharmaceutical formulation comprises the chimeric G250 antibody.

12. The method according to claim 10, wherein the pharmaceutical formulation comprises the chimeric G250 antibody in radiolabeled form.

13. The method according to claim 10, wherein the pharmaceutical formulation comprises a chimeric G250 antibody coupled to a cytokine such as IL-2, TNF and/or GM-CSF.

14. Use of a cell according to any one of claims 1-7 for the manufacture of a G250 antibody.

## Patentansprüche

1. Hybridomazelle DSM ACC 2526, die den monoklonalen Antikörper G250 produziert.

2. Verwendung der Hybridomazelle gemäß Anspruch 1 für die Herstellung einer Nachkommenzelle, die den monoklonalen Antikörper G250 produziert.

3. Verwendung gemäß Anspruch 2, worin die Nachkommenzelle durch Transfer von genetischem Material, welches für den Antikörper G250 oder zumindest eine Antigen-bindende Stelle davon kodiert, in eine Rezeptorzelle erhalten worden ist.

4. Verwendung gemäß Anspruch 2 oder 3, worin die Nachkommenzelle einen chimären Antikörper, einen humanisierten Antikörper, einen vollhumanisierten Antikörper, einen bispezifischen Antikörper, einen single-chain-Antikörper oder F(ab')₂, Fab' oder Fab Antikörperfragmente produziert.

5. Verwendung gemäß einem der Ansprüche 2 bis 4, worin die Nachkommenzelle eine prokaryotische Zelle ist.

6. Verwendung gemäß Anspruch 5, worin die Nachkommenzelle eine E. Coli-Zelle ist.

7. Verwendung gemäß einem der Ansprüche 2 bis 4, worin die Nachkommenzelle eine eukaryotische Zelle ist.

8. Verwendung gemäß Anspruch 7, worin die Nachkommenzelle eine Hefezelle, eine Myelomzelle oder eine CHO-Zelle ist.

9. Verfahren zur Herstellung eines Antikörpers G250, umfassend:
das Kultivieren einer Zelle gemäß einem der Ansprüche 1-7 unter geeigneten Bedingungen, bei denen der Antikörper hergestellt wird und das Gewinnen des Antikörpers aus der Zelle und/oder aus dem Kulturmedium.

10. Verfahren gemäß Anspruch 9 zur Verwendung für die Herstellung einer pharmazeutischen Zusammensetzung, die den Antikörper G250 als einen Wirkstoff umfasst.

11. Verfahren gemäß Anspruch 10, worin die pharmazeutische Zusammensetzung den chimären Antikörper G250 umfasst.

12. Verfahren gemäß Anspruch 10, worin die pharmazeutische Zusammensetzung den chimären Antikörper G250 in radiomarkierter Form umfasst.

13. Verfahren gemäß Anspruch 10, worin die pharmazeutische Zusammensetzung einen chimären Antikörper G250, der an ein Cytokin, wie zum Beispiel IL-2, TNF und/oder GM-CSF, gebunden ist, umfasst.

14. Verwendung einer Zelle gemäß einem der Ansprüche 1-7 zur Herstellung eines Antikörpers G250.

## Revendications

1. Cellule d'hybridome DSM ACC 2526 qui produit l'anticorps monoclonal G250.

2. Utilisation de la cellule d'hybridome selon la revendication 1 pour la préparation d'une cellule progénitrice qui produit l'anticorps monoclonal G250.

3. Utilisation selon la revendication 2, dans laquelle ladite cellule progénitrice a été obtenue par transfert de matériel génétique codant pour l'anticorps G250 ou au moins le site de liaison à l'antigène de celui-ci dans une cellule réceptrice.

4. Utilisation selon la revendication 2 ou 3, dans laquelle ladite cellule progénitrice produit un anticorps chimérique, un anticorps humanisé, un anticorps entièrement humanisé, un anticorps bispécifique, un anticorps à chaîne unique ou des fragments d'anticorps F(ab')₂, Fab' ou Fab.

5. Utilisation selon l'une quelconque des revendications 2 à 4, dans laquelle ladite cellule progénitrice est une cellule procaryote.

6. Utilisation selon la revendication 5, dans laquelle ladite cellule progénitrice est une cellule de E. coli.

7. Utilisation selon l'une quelconque des revendications 2 à 4, dans laquelle ladite cellule progénitrice est une cellule eucaryote.

8. Utilisation selon la revendication 7, dans laquelle ladite cellule progénitrice est une cellule de levure, une cellule de myélome ou une cellule CHO.

9. Procédé de production d'un anticorps G250 comprenant :
de cultiver une cellule selon l'une quelconque des revendications 1 à 7 dans des conditions appropriées, dans lequel l'anticorps est produit et d'obtenir l'anticorps à partir de la cellule et/ou du milieu de culture.

10. Procédé selon la revendication 9, utilisé pour produire une formulation pharmaceutique qui comprend l'anticorps G250 comme agent actif.

11. Procédé selon la revendication 10, dans lequel la formulation pharmaceutique comprend l'anticorps chimérique G250.

12. Procédé selon la revendication 10, dans lequel la formulation pharmaceutique comprend l'anticorps chimérique G250 sous une forme radio-marquée.

13. Procédé selon la revendication 10, dans lequel la formulation pharmaceutique comprend un anticorps chimérique G250 couplé à une cytokine telle que IL-2, TNF et/ou GM-CSF.

14. Utilisation d'une cellule selon l'une quelconque des revendications 1 à 7 pour la fabrication d'un anticorps G250.
